# EUROPÄISCHE PATENTSCHRIFT

(11) **EP 0 806 186 B1**
(45) Veröffentlichungstag und Bekanntmachung des Hinweises auf die Patenterteilung: **18.09.2002**
(21) Anmeldenummer: 97107229.3
(22) Anmeldetag: 30.04.1997
(51) Int. Cl.: A61B 19/00, A61F 11/00

(54) **Fixierbares Positioniersystem für die feste, spielfreie Anbindung an den menschlichen Schädel**
Connectable positioning system for the firm fixation without play to the human skull
Système de positionnement connectable pour la montage fixe sans jeu au crâne humain

(30) Priorität: 10.05.1996 DE 19618945
(43) Veröffentlichungstag der Anmeldung: 12.11.1997
(73) Patentinhaber: PHONAK AG, 8712 Stäfa (CH)
(72) Erfinder: Leysieffer, Hans, Dr. Dipl. Ing., 82024 Taufkirchen (DE); Lehner, Rolf, Dipl. Ing., 73734 Esslingen (DE)
(74) Vertreter: Schwan, Gerhard, Dipl.-Ing.

(56) Entgegenhaltungen:
- WO-A-95/22297
- DE-B- 1 242 792
- GB-A- 2 164 856
- US-A- 5 201 742
- US-A- 5 263 956

## Beschreibung

Die Erfindung bezieht sich auf ein fixierbares Positioniersystem für die feste, spielfreie Anbindung an den menschlichen Schädel, insbesondere zur nichtinvasiven Ankopplung eines aktorischen oder sensorischen Instruments an die extrakorporale Seite des Trommelfells.

Aufgrund der Tatsache, daß durch handgeführte medizinische Instrumente teilweise beträchtliche Relativbewegungen zwischen Instrument und körperfesten Zielstrukturen auftreten können und daß diese Relativbewegungen zum Teil mit erheblichen Risiken für den Patienten verbunden sind, ist es wünschenswert, derartige Instrumente einerseits körperfest zu verankern, andererseits aber nach erfolgter körperfester Verankerung in gewissen Grenzen räumlich führen zu können. Zudem soll die räumliche Lage zwischen positioniertem Instrument und körperfestem Zielort fixierbar sein, damit eine Positionsänderung des Werkzeugs während nachfolgender chirurgischer, therapeutischer oder diagnostischer Arbeitsschritte vermieden wird.

Die körperfeste Fixierung von Vorrichtungen zur Aufnahme und räumlichen Positionierung geeigneter Instrumente am menschlichen Schädel, ist jedoch für eine Vielzahl von Eingriffen kompliziert, da der menschliche Schädel nur wenige Strukturen aufweist, an denen geeignete Halterungen reversibel festzumachen sind. Um Relativbewegungen zwischen dem Körper des Patienten und geführten Instrumente zu vermeiden, besteht grundsätzlich die Möglichkeit, Patient und Werkzeug in eine feste mechanische Verbindung zu bringen.

Angesichts der außerordentlich kleinen und empfindlichen anatomischen Strukturen im menschlichen Körper ist das längere (mehr als wenige Sekunden) dauernde, handgeführte Beibehalten der Instrumentenposition nahezu unmöglich oder erfordert vom Arzt bzw. Operateur einen erheblichen Kraft- und Konzentrationsaufwand. Viele Eingriffe im Körper, vor allem im Schädelbereich, erfordern aber gerade eine über längere Zeiträume fixierbare, zielpunktgerichtete Positionierung der verwendeten Instrumente.

Aus dem Stand der Technik sind bereits zwei verschiedene Kopfhalterungen bekannt. Die sogenannte *Mayfield-Klemme* wird fest am Kopfende des Operationstisches mit einem Stativ verankert. Der Kopf des Patienten wird in die tischfeste Aufnahme eingelegt und mittels dreier konischer Gewindedorne, die durch die Haut auf den Schädelknochen drücken, dort eingespannt. Die Prozedur ist insgesamt schmerzhaft und kann für neurochirurgische Eingriffe nur in Lokalanästhesie oder Vollnarkose durchgeführt werden, wobei für letztere in der Regel ein stationärer Aufenthalt in einer HNO-Klinik erforderlich ist.

Von der Firma *Radionics, Wiesbaden* wurde ein Rahmen auf den Markt gebracht, der mit vier konischen Gewindedornen am Schädel befestigt wird. Dieser Rahmen kann vom Patienten kurzfristig mobil getragen oder bei Bedarf ebenfalls am Operationstisch fixiert werden. Der Rahmen dient als Koordinatenbasis für stereotaktische Zieloperationen im Schädel. Auch bei dieser bekannten Vorrichtung ist das Einspannen des Schädels mit den Metalldornen schmerzhaft, und es sollte unter örtlicher Betäubung erfolgen.

Beide beschriebene Halterungen dienen dazu, Relativbewegungen zwischen Kopf und Operationstisch auszuschließen. Mit ebenfalls tischfest fixierten Werkzeugen kann dann die erforderliche Präzision zwischen Werkzeug und Kopf hergestellt werden. Solche Systeme sind technisch aufwendig, an den Operationssaal gebunden und weisen zudem den Nachteil auf, daß der Patient zur Einspannung des Schädels in der Haltevorrichtung betäubt sein muß, da derartige Halterungen mit relativ hohen punktförmig eingeleiteten Druckkräften arbeiten.

Die Erfindung stellt sich die Aufgabe, die aus dem Stand der Technik bekannten Nachteile zu vermeiden. So soll insbesondere ein -vorzugsweise relativ kleines und leichtesmehrachsiges Positioniersystem geschaffen werden, das in Kombination mit einer geeigneten Halterung, insbesondere einer Kopfhalterung, direkt am Schädel des Patienten befestigt werden kann, ohne dabei eine größere Basis wie z.B. einen Operationstisch einzubeziehen. Das Positioniersystem samt der jeweils für den Einsatzzweck gewählten Halterung soll sich ohne zusätzliche Hilfsmittel einfach und problemlos am Kopf des Patienten befestigen lassen.

Diese Aufgabe wird gelöst durch ein fixierbares Positioniersystem für die feste, spielfreie Anbindung an den menschlichen Schädel, insbesondere zur nichtinvasiven Ankopplung eines aktorischen oder sensorischen Instruments an die extrakorporale Seite des Trommelfells mit:
- einer an dem Schädel befestigbaren Halterung;
- einer an der Halterung angebrachten Basis;
- einer Linearantriebsanordnung, die eine Linearführung mit einem daran angebrachten Innengewindeteil sowie eine achsparallel zu der Linearführung angeordnete, mit Bezug auf die Linearführung manuell drehbare Gewindespindel aufweist;
- einer in der Linearführung geführten Aufnahmevorrichtung für ein eine Längsachse aufweisendes therapeutisches oder diagnostisches Instrument; und
- einem zwischen der Basis und der Linearführung angeordneten, manuell positionierbaren und über einen Klemmechanismus fixierbaren Kugelgelenk;
- wobei die Längsachse des Instruments gegenüber der Achse der Linearführung und gegenüber dem Kugelgelenk seitlich versetzt ist und wobei die Konstruktion und die geometrischen Abmessungen des Positioniersystems derart gestaltet sind, daß die bedienende Person stets mit bloßem Auge oder bei Verwendung eines Mikroskopes freie Sicht auf mindestens das freie Wirkende des Instruments sowie den Zielpunkt im Körper behält.

Das erfindungsgemäße Positioniersystem erlaubt die Aufnahme und spielfreie Befestigung bzw. den einfachen Wechsel eines beliebigen Instruments, und es ermöglicht darüber hinaus dem Arzt, mittels mehrerer Achsen dieses Instrument präzise und frei von Relativbewegungen zu Strukturen am oder im menschlichen Körper hinzuführen.

Das schädelfest fixierte Positioniersystem dient mit seiner Aufnahme für beliebige aktorische, sensorische, mechanische oder optische Instrumente als "künstliche, tremorfreie Hand" des Arztes oder Chirurgen, der mittels mehrerer feinmechanischer Achsen das freie Wirkende des Instruments im Körper zu einem körperfesten Zielpunkt positionieren kann, ohne daß nennenswerte risikobehaftete Relativbewegungen auftreten.

Durch das vorliegend vorgeschlagene klemmbare Positioniersystem, insbesondere durch die nachfolgend genannten Ausführungen, wird eine Vielzahl von Vorteilen erreicht. Es ergibt sich ein einfacher, kompakter Aufbau aus relativ wenigen Bauteilen, die selbst wiederum einfach aufgebaut und konstruiert sein können. Dies gewährleistet auch ein hohes Maß an gerätetechnischer Sicherheit beim bestimmungsgemäßen Gebrauch für Anwender, Patienten und Dritte. Auch äußere Einflüsse wie z.B. Erschütterungen, Temperatur-, Lage und Druckwechsel können die Funktion des Systems nicht negativ beeinflussen.

Bevorzugte Ausführungsformen der Erfindung sind in den Unteransprüchen angegeben.

Soll ein körperfester Zielpunkt angefahren und danach die Position des Positioniersystems gehalten werden, ist ein Feststellmechanismus bzw. die selbsttätige Feststellung für die rotatorischen bzw. translatorischen Achsen des Systems von Vorteil. Insbesondere sind zur Vermeidung einer selbsttätigen Verstellung des in der Aufnahmevorrichtung sitzenden Instruments das Innengewindeteil und die Gewindespindel selbsthemmend ausgeführt. Dabei kann mit der Gewindespindel ein Drehknauf verbunden sein, der bei einer manuellen Verdrehung für das axiale und selbsthemmende Verfahren der Linearantriebsanordnung sorgt.

Um für ein einfaches und schnelles Fixieren aller drei rotatorischen Freiheitsgrade des Kugelgelenks in ihrer aktuellen Lage zu sorgen, ist vorzugsweise eine Klemmschraube vorgesehen, wobei zusätzlich dafür gesorgt sein kann, daß die aktuelle Position der rotatorischen Freiheitsgrade bei gelöster Klemmung des Kugelgelenks durch Reibkräfte gesichert ist und nach dem Schließen des Klemmung dauerhaft beibehalten wird.

Je nach Anwendung kann das Positioniersystem auf mehrere Arten am Schädel des Patienten befestigt werden. Für invasive, intraoperative Einsätze wird es z.B. fest mit einem Wundsperrer verbunden. Für nichtinvasive Therapie- oder Diagnoseanwendungen im Ohrbereich ist es fester Bestandteil eines Kopfhalters. Durch diese Halterungen (Wundsperrer bzw. Kopfhalter) werden Relativbewegungen zwischen den am oder im Körper befindlichen, teilweise mikroskopisch kleinen Zielstrukturen und dem im Positioniersystem fest eingespannten Instrument (Sensor, Aktor, chirurgisches Werkzeug) auf ein Minimum reduziert. Zudem ist für die Befestigung der Halterung am Schädel bei chirurgischer Verwendung kein zusätzlicher chirurgischer Eingriff als die ohnehin erforderliche Körperöffnung (Haut- oder Gewebeschnitt) erforderlich.

Bei Verwendung des Positioniersystems mit einem Kopfhalter für nichtinvasive Anwendungen läßt sich das System schmerzfrei und unter Vermeidung von chirurgischen Maßnahmen (Gewebe- oder Hautschnitte etc.) fest am Schädel fixieren. Insbesondere kann der Kopfhalter auf dessen Arbeitsseite mit einem trichterförmigen Halteelement zur Fixierung im Gehörgang versehen sein, wobei in diesem Fall das freie Wirkende des Instrumentes zum Zielort am oder im Körper durch das trichterförmige Halteelement hindurchführbar ist. Insbesondere zur beidseitigen schmerzfreien Befestigung im Ohrbereich des Schädels kann der Kopfhalter auf der der Arbeitsseite gegenüberliegenden Seite ein die Ohrmuschel umschließendes, weiches Formteil aufweisen. Diese Formteil dient dann als zweite Auflagefläche für den Kopfhalter, es kann jedoch auch als einseitiger Kopfhörer oder als Schallschutz ausgebildet sein.

Um für eine möglichst optimale Anpassung der Einspannweite des Kopfhalters zwischen Arbeits- und Gegenseite zu sorgen, können ferner ein Gewindetrieb und ein diesem zugeordneter Drehknauf vorgesehen sein, mittels dessen sich die Einspannweite stufenlos und selbsthemmend verstellen läßt.

Ein bevorzugtes Instrument für eine nichtinvasive Anwendung des erfindungsgemäßen Positioniersystems ist ein piezoelektromechanischer Hörgerätewandler zur vibratorischen Stimulation des Trommelfells an dessen extrakorporaler Seite.

Als Körpereintrittsöffnungen, um das im Positioniersystem festgehaltene Instrument in den Körper präzise einzubringen und dort zu positionieren, kommen z.B. der Gehörgang, die eröffnete, hinter dem Gehörgang gelegene Mastoidhöhle, ein Durchbruch im Schädelknochen oder ein Hautschnitt über der Wirbelsäule in Betracht. Dadurch sind mikrochirurgische Manipulationen z.B. auf dem Trommelfell, an der Gehörknöchelchenkette, im Innenohr, im Vestibularorgan oder im Gewebe von Gehirn oder Rückenmark möglich. Wird als Instrument z.B. eine mikrochirurgische Glasfaser verwendet, kommen darüber hinaus auch laserchirurgische Operationen von kleinen Tumoren, Mikrokoagulationen oder Gewebeverödungen in Betracht. Bei Einkopplung eines meßtechnischen Lasers können Schwingungen z.B. der Gehörknöchelchenkette oder des Trommelfells berührungsfrei gemessen werden.

Bei Kombination des oben beschriebenen Positioniersystems mit einem Wundsperrer für intraoperative Anwendungen
- ist die Basis des Positioniersystems mechanisch fest mit dem Wundsperrer verbunden,
- ist das Positioniersystem so am Wundsperrer angeordnet, daß die einzelnen Bedienelemente vom Körper des Patienten wegzeigen, um sie problemlos manuell bedienen zu können,
- ist das Instrument in der Aufnahme eines Schlittens so angeordnet und fixiert, daß das freie Wirkende des Instruments in das Körperinnere zum körperfesten Zielort zeigt,
- sind die Bedienelemente des Positioniersystems so angeordnet, daß der bedienende Arzt stets noch freie Sicht auf das Instrument, dessen freies Wirkende im Körper und den körperfesten Zielort hat,
- ist die Kombination von Wundsperrer und Positioniersystem so angeordnet, daß auch relativ kleine, chirurgisch ohnehin erforderliche Körperöffnungen genügen, um das freie Wirkende des Instruments auch unter mikroskopischer Kontrolle zu führen,
- sind Positioniersystem und Wundsperrer mit einfachen Mitteln zu reinigen und zu sterilisieren.

Vorzugsweise ist das Positioniersystem mit einem Kopfhalter für nichtinvasive Anwendungen ausgestattet. Dabei
- ist die Basis des Positioniersystems mechanisch fest mit dem Kopfhalter verbunden,
- ist das Positioniersystem so am Kopfhalter angeordnet, daß die einzelnen Bedienelemente vom Körper wegzeigen, um sie problemlos manuell bedienen zu können,
- ist das Instrument in der Aufnahme eines Schlittens so angeordnet, daß das freie Wirkende des Instruments in das Körperinnere zum Zielort zeigt,
- ist das auf der Arbeitsseite des Kopfhalters gelegene Halteelement so ausgebildet, daß es sich mit seiner Außenfläche konisch in den Gehörgang einführen läßt, und beim Schließen des Kopfhalters fest in diesen eingedrückt wird,
- weist das auf der Arbeitsseite des Kopfhalters gelegene Halteelement eine ebenfalls konische Innenbohrung auf, die als Durchtritt für das freie Wirkende des im Schlitten eingelegten Instruments sowie für die optische Achse eines Mikroskopes dient,
- ist die Kombination von Kopfhalter und Positioniersystem so ausgelegt, daß auch relativ kleine Körperöffnungen wie z.B. der Gehörgang genügen, um das freie Wirkende des Instruments auch unter mikroskopischer Kontrolle in den Körper einzubringen,
- ist der Kopfhalter so ausgeführt, daß er durch beidseitigen Druck der Halteelemente am Kopf zwar fest, aber dennoch schmerzfrei und nichtinvasiv fixiert werden kann,
- ist der Kopfhalter so ausgeführt, daß durch einen Betätigungsmechanismus der Abstand der beiden Halteelemente durch den Patienten selbst oder durch den Arzt verändert werden kann,
- ist der gewählte Abstand zwischen den beiden Halteelementen durch Selbsthemmung gesichert,
- sind die beiden Halteelemente entweder als Ohrtrichter ausgebildet, oder ist der Gegenhalter, der nicht mit dem Positioniersystem kombiniert ist, als Gehörschutzkapsel ausgebildet, die auf die Ohrmuschel aufgesetzt wird, diese umschließt und dadurch nicht verrutschen kann,
- kann durch Verwendung einer Gehörschutzkapsel als Gegenhalter bei Bedarf das Gegenohr schallgeschützt werden,
- kann durch Verwendung eines einseitigen Kopfhörers als Gegenhalter bei Bedarf das Gegenohr mit der aktuellen Untersuchung dienenden Schallen beaufschlagt werden.

Die Erfindung wird nachfolgend unter Bezugnahme auf die beiliegenden Zeichnungen näher beschrieben. Es zeigen :
- FIG. 1: eine schematische Gesamtansicht des Positioniersystems nach der Erfindung,
- FIG. 2a: eine Schnittdarstellung des ungeklemmten, aber durch Federkraft in seiner Position gehaltenen Kugelgelenkes des Positioniersystems nach der Erfindung,
- FIG. 2b: eine Schnittdarstellung des geklemmten Kugelgelenkes des Positioniersystems nach der Erfindung,
- FIG. 3: eine schematische Gesamtansicht des Positioniersystems kombiniert mit einem Wundsperrer zur intraoperativen Anwendung,
- FIG. 4: eine schematische Gesamtansicht des Postioniersystems kombiniert mit einem Kopfhalter zur nichtinvasiven, diagnostischen bzw. therapeutischen Anwendung.

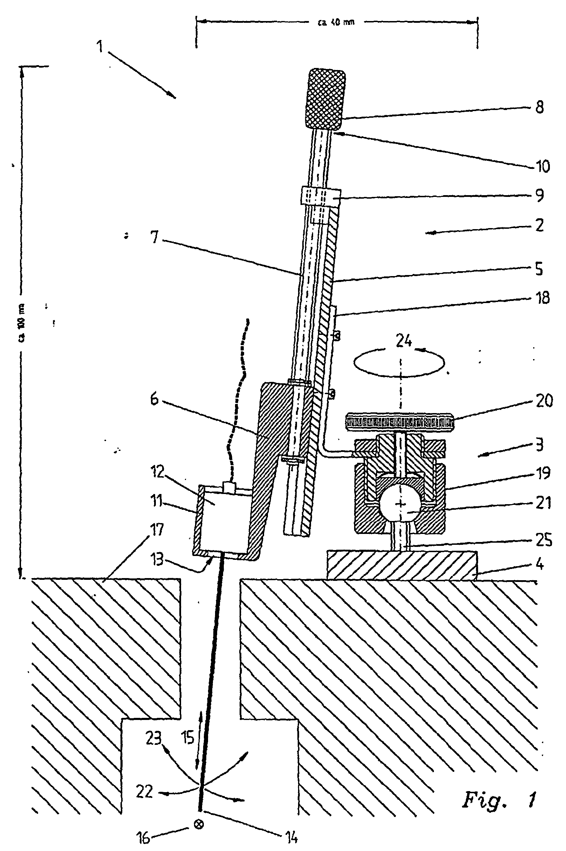

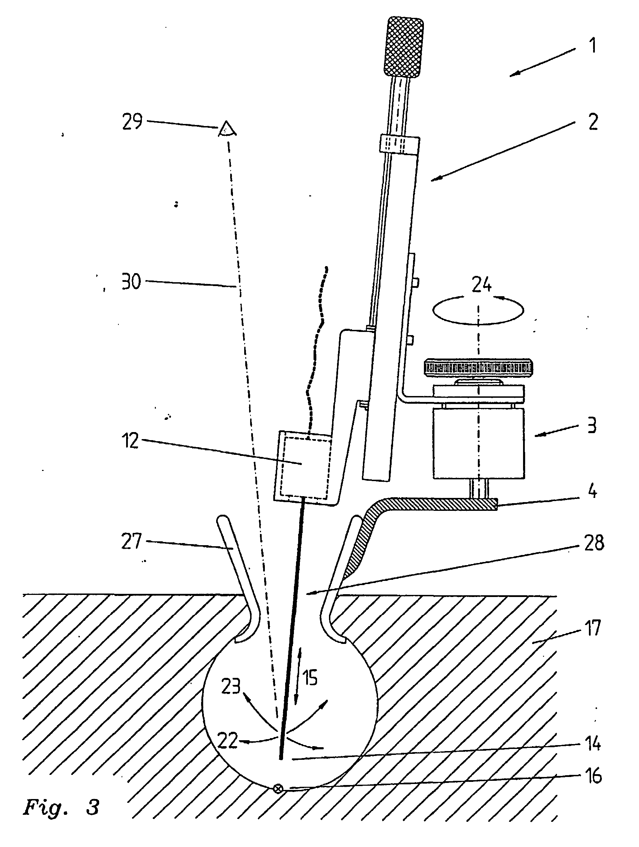

Das in den FIGN. 1 und 3 dargestellte Positioniersystem 1 setzt sich im wesentlichen aus einer Linearantriebsanordnung 2, einem klemmbaren Kugelgelenk 3 und einer Basis 4 zusammen.

In einer Linearführung (Führungsschiene) 5 der Linearantriebsanordnung 2 ist ein Schlitten 6 spielfrei geführt. Der Schlitten 6 ist mit dem einen Ende einer Gewindespindel 7 verbunden, die mit einem an der Linearführung 5 angebrachten Innengewindeteil 9 in Gewindeeingriff steht. Ein Drehknauf 8 ist mit dem anderen Ende der Gewindespindel 7 fest verbunden. Durch Drehen der Gewindespindel 7 mittels des Drehknaufs 8 läßt sich der Schlitten 6 entlang der Linearführung 5 verfahren. Die Steigungen der Gewindespindel 7 und des Innengewindeteils 9 sind selbsthemmend ausgelegt, das heißt, ein Verstellen des Schlittens 6 ist nur durch Drehen der Gewindespindel 7 möglich, während von dem Schlitten 6 auf die Gewindespindel 7 übertragene Kräfte nicht in der Lage sind, die Gewindespindel 7 zu drehen. Infolgedessen ist es ausgeschlossen, daß sich der Schlitten 6 entlang der Linearführung 5 aufgrund von z.B. Gravitationskräften ungewollt selbsttätig bewegt.

Der Verfahrweg des Schlittens 6 entlang der Linearführung 5 ist durch zwei Endanschläge begrenzt. Der eine Endanschlag wird von der dem Schlitten 6 zugewendeten Seite des Innengewindeteils 9 gebildet, der andere von der dem Innengewindeteil 9 zugewendeten Stirnfläche 10 des Drehknaufs 8. Das an der Linearführung 5 angebrachte Innengewindeteil 9 führt zum einen die Gewindespindel 7 parallel zu der Linearführung 5, verhindert zum anderen aber auch, daß durch zu weites Herausdrehen der Spindel 7 der Schlitten 6 aus der Linearführung 5 gleitet. Entsprechend verhindert die Stirnfläche 10 am unteren Ende des Drehknaufs 8 ein zu weites Eindrehen der Gewindespindel 7 und somit ein Herausgleiten des Schlittens 6 am gegenüberliegenden Ende der Linearführung 5.

Durch Drehbewegung am Drehknauf 8 wird entsprechend dem Gewindedrehsinn und der gewählten Steigung der Gewindespindel 7 und des Innengewindeteils 9 eine Axialverschiebung des Schlittens 6 in der Führung 5 der Linearantriebsanordnung 2 bewirkt. Der Schlitten 6 läßt sich somit entlang der Linearantriebsanordnung 2 zwischen den beiden Anschlägen 9 und 10 stufenlos verfahren, und er hält aufgrund der Selbsthemmung des Gewindetriebs seine momentane Position.

Der Schlitten 6 besitzt eine Aufnahme 11, in die das gewünschte Instrument 12 manuell und spielfrei eingesetzt bzw. daraus entnommen werden kann. Die Aufnahme 11 für das Instrument 12 weist einen Durchbruch 13 für das freie Wirkende 14 des darin eingelegten Instruments 12 auf. Die Längsachse des Instruments 12 ist sowohl gegenüber der Längsachse der Linearführung 5 als auch gegenüber dem Kugelgelenk seitlich versetzt. Das freie Wirkende 14 des eingelegten Instruments 12 ist somit bei Drehung des Drehknaufs 8 parallel zur Linearführung in axialer Richtung 15 zu einem körperfesten Zielpunkt 16 im Körper 17 positionierbar.

Die Linearantriebsanordnung 2 samt Gewindespindel 7, Drehknauf 8, Schlitten 6 und in der Aufnahme 11 eingelegtem und dort gehaltenem Instrument 12 ist mit einem Verbindungselement 18 mit einem Gehäuse 19 des klemmbaren Kugelgelenks 3 fest verbunden. Das Kugelgelenk 3 weist eine Kugel 21 auf, die über eine Säule 25 mit der Basis 4 in fester Verbindung steht und die mittels einer Klemmschraube 20 mit Bezug auf das Gehäuse 19 festgeklemmt werden kann.

Bei gelöster Klemmung des Kugelgelenks 3 läßt sich die gesamte Linearantriebsanordnung 2 um das Zentrum der ortsfest mit der Basis 4 verbundenen Kugel 21 in allen drei rotatorischen Freiheitsgraden des Raums 22, 23, 24 drehen.

Über die Basis 4 läßt sich das Positioniersystem 1 mit geeigneten Halterungen fest verbinden. Nach Befestigung einer dieser Halterungen am Schädel, Positionieren des an der Halterung befestigten Systems und nachfolgender Klemmung der Klemmschraube 20 ist somit eine exakte, spielfreie Positionierung des freien Wirkendes 14 zu einem körperfesten Zielpunkt 16 möglich, wobei eventuell risikobehaftete Relativbewegungen zwischen Körper und freiem Wirkende 14 des Instruments vermieden werden.

Durch Lösen der Klemmschraube 20 des Kugelgelenks 3 lassen sich das Verbindungselement 18 sowie die daran befestigte Linearantriebsanordnung 2 und das im Schlitten 6 eingelegte Instrument 12 samt seinem freien Wirkende 14 um das Zentrum der Kugel 21 des Kugelgelenks 3 entsprechend allen drei rotatorischen Freiheitsgraden im Raum 22, 23, 24 drehen. Die gezeigte Kombination aus einem klemmbaren Kugelgelenk 3 und der daran fest angebrachten Linearantriebsanordnung 2 ermöglicht zusammengefaßt die vierachsige Positionierung des freien Wirkendes 14 des gewählten Instruments 12 entsprechend dem translatorischen 15 und den drei rotatorischen Freiheitsgraden 22, 23, 24 zu einem beliebigen, körperfesten Zielpunkt 16.

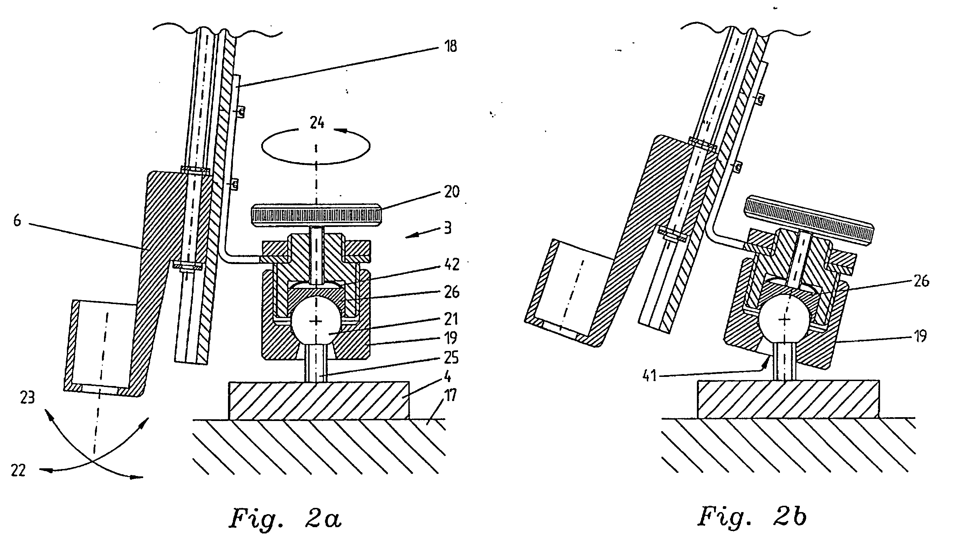

Die Funktionsweise des klemmbaren Kugelgelenks 3 wird anhand der FIGN. 2a und 2b verdeutlicht. In FIG. 2a ist die Klemmung des Kugelgelenks 3 gelöst. Die räumliche Position der drei Drehachsen 22, 23, 24 wird durch Federvorspannung gehalten. In FIG. 2b ist die Linearantriebsanordnung zur Seite geschwenkt und das Kugelgelenk geklemmt. Die Sicherung der Momentanposition erfolgt durch Federkraftvorspannung und zusätzlich durch Axialkraft der Klemmschraube 20 auf die Kugelfläche.

Durch einen konischen Durchbruch 41 an der Unterseite des Gehäuses 19 des Kugelgelenks 3 führt die Säule 25 zur Basis 4. Kugel 21, Säule 25 und Basis 4 bilden somit eine starre Einheit. Die Klemmschraube 20 wirkt über einen verschiebbaren Kugelsitz 26 auf die Außenfläche der Kugel 21. In FIG. 2b ist dargestellt, wie die Kugel 21 beim Anziehen der Klemmschraube 20 zwischen den Kontaktflächen des Kugelsitzes 26 und den entsprechenden Gegenflächen des Gehäuses 19 fest eingespannt wird. Die gewählte Raumposition der drei rotatorischen Freiheitsgrade 22, 23, 24 ist somit fest fixiert. Beim in FIG. 2a dargestellten Lösen der Klemmschraube 20 soll umgekehrt die aus der Klemmung freigegebene Kugel nicht ein Umkippen des gesamten Positioniersystems zulassen, sondern die gewählte Position soll weiterhin reibschlüssig beibehalten werden. Dies wird erreicht, indem auf den Kugelsitz 26 auch nach dem Lösen der Klemmschraube 20 ein axial vorgespanntes Federelement 42 wirkt, das bei entsprechend vorgewählter Vorspannung die räumliche Position des Kugelgelenks unter Reibschluß sichert, aber manuelle Bewegungen des Gesamtsystems zuläßt.

Die gesamte Linearantriebsanordnung 2 samt eingelegtem Instrument 12 und Gehäuse 19 des Kugelgelenks 3 läßt sich somit um das Zentrum der Kugel 21 in allen drei rotatorischen Freiheitsgraden 22, 23, 24 schwenken, wobei die Reibung des vorgespannten Federelements 42 auf die Kugelfläche auch bei gelöster Klemmschraube 20 ein Verkippen des Systems vermeidet.

Die Hauptabmessungen des gesamten Systems betragen beispielsweise als Gesamthöhe über der Körperoberfläche ca. 100 mm und als Gesamtbreite ca. 40 mm.

In FIG. 3 ist eine Kombination aus dem beschriebenen Positioniersystem 1 und einem chirurgischen Wundsperrer 27 zur intraoperativen Anwendung dargestellt. Dabei wird das Positioniersystem 1 samt der Linearantriebsanordnung 2 und dem klemmbaren Kugelgelenk 3 mit seiner Basis 4 fest mit einem beliebigen Wundsperrer 27 verbunden. Nach Einsetzen des Wundsperrers 27 in eine Körperöffnung 28, die z.B. durch einen chirurgischen Gewebeschnitt realisiert ist, und nachfolgendem Aufspreizen des Wundsperrers 27 in der Körperöffnung 28, wird der Wundsperrer 27 samt daran befestigtem Positioniersystem 1 mit dem Körper fest verbunden.

Durch Manipulation der vier Bewegungsachsen 15, 22, 23 und 24 läßt sich intraoperativ das freie Wirkende 14 eines beliebigen, in das Positioniersystem fest eingespannten Instruments 12 präzise zu einem beliebigen im Körper 17 befindlichen Zielort 16 führen. Falls die Positionierung an Strukturen erfolgen soll, die mit bloßem Auge nicht mehr sichtbar sind, kann die Position des freien Wirkendes in der Körperöffnung visuell durch z.B. ein Mikroskop kontrolliert werden. Dabei ist das Positioniersystem 1 so ausgelegt und an dem Wundsperrer 27 angeordnet, daß die optische Achse 30 des Mikroskopes bzw. des bloßen Auges 29 nicht vom Positioniersystem 1 selbst oder dessen Bestandteilen verdeckt wird.

Bevorzugte Körperöffnungen 28, bei denen das Positioniersystem 1 intraoperativ mit einem geeigneten Wundsperrer 27 angewendet werden kann, sind z.B. Schnitte in Weichteilgeweben, der äußere Gehörgang, die Mastoidhöhle oder Durchbrüche im Schädelknochen bei neurochirurgischen Eingriffen.

Dementsprechend sind bevorzugte körperfeste Zielorte 16, auf die intraoperativ das freie Wirkende 14 des positionierbaren Instruments 12 gerichtet werden soll, u.a. mikrochirurgische Strukturen innerhalb des Schädels, im äußeren Gehörgang, auf dem Trommelfell, in den geöffneten Räumen des Mittelohres (Hammer, Amboß, Steigbügel), an der Knochenwand zwischen Mittel- und Innenohr (*Promontorium*), an dem flüssigkeitsgefüllten Innenohr, am Hörnerven, im Gleichgewichtsorgan oder in der Augenhöhle.

Geeignete Instrumente 12 für die Anwendung des Positioniersystems 1 in Verbindung mit Wundsperrern 27 sind u.a. piezoelektrische Hörgerätewandler zur elektromechanischen vibratorischen Stimulation der Gehörknöchelchenkette, Erregerspulen zur elektromagnetischen Stimulation von kettenfest fixierten Permanentmagneten, Lichtleiter zur Führung von chirurgischem Laserlicht (z.B. zum Schneiden, Bohren, Koagulieren oder Veröden von Gewebe oder Knochenstrukturen), Lichtleiter zur Führung von meßtechnischem Laserlicht (Laser-Doppler-Vibrometrie), flexible Miniaturendoskope zur Inspektion im gesamten Schädelbereich, Sondenmikrophone und kleine Schallquellen zur intraoperativen Audiometrie (Hörschwellenbestimmung, Messung otoakustischer Emissionen) sowie Elektroden zur elektrocochleographischen Ableitung von Körperpotentialen wie z.B. Summenaktionspotential (SAP) oder Mikrophonpotential (MP).

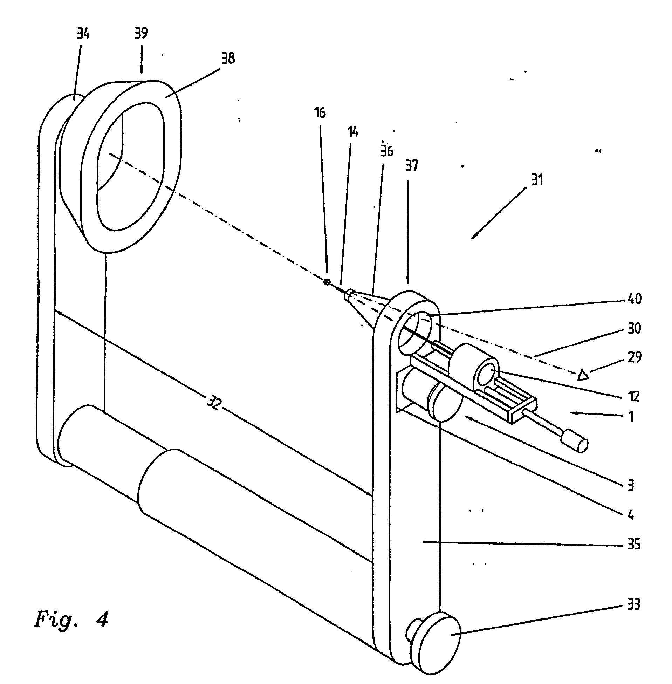

In FIG. 4 ist als bevorzugte Ausführungsform eine Kombination aus dem Positioniersystem 1 und einem Kopfhalter 31 zur nichtinvasiven, diagnostischen oder therapeutischen Anwendung von schädelfest verankerten Instrumenten dargestellt. In der gezeigten Ausführung ist das Positioniersystem 1 mit seiner Basis 4 mit einem Kopfhalter 31 fest verbunden. Die Öffnungsweite 32 des Kopfhalters beträgt vorzugsweise etwa 200 mm, und sie läßt sich über einen Drehknopf 33 und einen innenliegenden Gewindetrieb beliebig und spielfrei verstellen, indem zwei Aufnahmearme 34 und 35 aufeinander zu (Schließen) bzw. von einander weg (Öffnen) bewegt werden. Der Drehknopf 33 zum Einstellen der Öffnungsweite 32 kann dabei entweder vom Träger der Kopfhalterung 31 selbst oder einer qualifizierten Fachkraft (Arzt, Assistent) bedient werden, um den Kopfhalter 31 am Kopf des Patienten durch beidseitiges Klemmen zu befestigen. Das Positioniersystem 1 ist mit seiner Basis 4 an einem der beiden Aufnahmearme 35 fest angebracht. Diese Seite wird als Arbeitsseite 37 des Kopfhalters bezeichnet. Ein konisches Halteelement 36 kann z.B. wie ein Ohrtrichter ausgeführt sein, der bei Bedarf zum Ausgleichen geringer räumlicher Winkel im Aufnahmearm 35 kardanisch gelagert sein kann. Es wird unter visueller Kontrolle, bei Bedarf unter Zuhilfenahme eines Mikroskops, in den äußeren Gehörgang des Trägers (Patienten) eingeführt.

Das konische Halteelement 36 besitzt zudem eine konische Innenbohrung 40, die Raum für das freie Wirkende 14 des im Positioniersystem 1 eingespannten Instruments 12 und die visuelle Kontrolle bietet.

Am gegenüberliegenden Aufnahmearm 34 auf der Gegenseite 39 des Kopfhalters 31 ist wahlweise ein zweiter konischer Halter 36 oder ein halbschalenförmiges Kapselelement 38 angebracht, das dort ebenfalls in den Gehörgang eingeführt oder über die Ohrmuschel des Gegenohres gelegt wird.

Bei Verwendung eines Kapselelements 38, wie es in FIG. 4 dargestellt ist, wird ein Teil der Vorspannkraft aufgrund des Schließens der Öffnungsweite 32 großflächig auf den die Ohrmuschel umgebenden Schädelknochenbereich übertragen. Punktförmige Druckkrafteinleitungen und ein damit verbundenes unerwünschtes Druckgefühl werden somit vermieden, und die zum Haltern aufgebrachte Kraft wird auf ein großflächiges Hautareal verteilt.

Nach Einführen des konischen Halteelements 36 in den äußeren Gehörgang der Arbeitsseite 37 und nachfolgendem Aufsetzen des Kapselelementes 38 auf der Ohrmuschel der Gegenseite 39 können durch vorsichtiges Verringern der Öffnungsweite 32 des Kopfhalters 31 die beiden Halteelemente, d.h. Halteelement 36 und Kapselelement 38, gegeneinander angenähert werden, bis der gesamte Kopfhalter 31 am Schädel des Patienten festgespannt ist. Durch Verformen des Kapselelementes 38 und Verkeilen des konischen Halteelements 36 im äußeren Gehörgang wird ein sicherer Sitz des gesamten Kopfhalters 31 am Schädel des Patienten gewährleistet. Durch die konische Innenbohrung 40 im konischen Halteelement 36 kann nach Festklemmen des Kopfhalters 31 am Schädel des Patienten das freie Wirkende 14 des im Positioniersystem 1 befestigten Instruments 12 spielfrei und unter Vermeidung von Relativbewegungen zwischen Schädel und schädelfesten Zielpunkten 16 positioniert werden. Über die bereits beschriebene Klemmeinrichtung des Positioniersystems läßt sich die eingestellte Position des Positioniersystems fixieren (vgl. FIG. 2a und FIG. 2b).

Eine bevorzugte Körperöffnung 28, bei der das Positioniersystem 1 nichtinvasiv bzw. minimalinvasiv mit einem geeigneten Kopfhalter 31 angewendet werden kann, ist der äußere Gehörgang des Menschen.

Dementsprechend sind bevorzugte körperfeste Zielorte 16, auf die das freie Wirkende 14 des positionierbaren Instrumentes 12 nichtinvasiv gerichtet werden soll, u.a. mikrochirurgische Strukturen im Gehörgang selbst, auf oder im Trommelfell, insbesondere der Umbo, oder der direkt mit der Trommelfell-Innenseite verwachsene Hammergriff (*Manubrium*), der den am weitesten außenliegenden, durch den Gehörgang nichtinvasiv erreichbaren Punkt der Gehörknöchelchenkette darstellt.

Bei lokaler, minimalinvasiver Anwendung dieser zweiten Ausführungsform ist nach Eröffnen des Trommelfells darüber hinaus eine Positionierung des Instruments hin zu den anatomischen Strukturen des luftgefüllten Mittelohres (Hammer, Amboß, Steigbügel) oder der Knochenwand zwischen Mittel- und Innenohr (*Promontorium*) möglich.

Für die nichtinvasive Anwendung des Positioniersystems 1 in Verbindung mit geeigneten Kopfhaltern 31 eignen sich gleichermaßen alle unter Bezugnahme auf die erste bevorzugte Ausführungsform beschriebenen Instrumente. Insbesondere kommt die exakte Ankopplung eines elektromechanischen Wandlers an den Umbo in Betracht, beispielsweise für eine elektromechanische Hörprüfung im Hinblick auf eine Implantation eines elektromagnetischen Mittelohrstimulators.

## Patentansprüche

1. Fixierbares Positioniersystem für die feste, spielfreie Anbindung an den menschlichen Schädel, insbesondere zur nichtinvasiven Ankopplung eines aktorischen oder sensorischen Instruments an die extrakorporale Seite des Trommelfells mit:
- einer an dem Schädel befestigbaren Halterung (27, 31);
- einer an der Halterung (27, 31) angebrachten Basis (4);
- einer Linearantriebsanordnung (2), die eine Linearführung (5) mit einem daran angebrachten Innengewindeteil (9) sowie eine achsparallel zu der Linearführung (5) angeordnete, mit Bezug auf die Linearführung manuell drehbare Gewindespindel (7) aufweist;
- einer in der Linearführung (5) geführten Aufnahmevorrichtung (6, 11) für ein eine Längsachse aufweisendes therapeutisches oder diagnostisches Instrument (12); und
- einem zwischen der Basis (4) und der Linearführung (5) angeordneten, manuell positionierbaren und über einen Klemmechanismus (20, 26, 42) fixierbaren Kugelgelenk (3);
- wobei die Längsachse des Instruments (12) gegenüber der Achse der Linearführung (5) und gegenüber dem Kugelgelenk (3) seitlich versetzt ist und wobei die Konstruktion und die geometrischen Abmessungen des Positioniersystems derart gestaltet sind, daß die bedienende Person stets mit bloßem Auge oder bei Verwendung eines Mikroskopes freie Sicht auf mindestens das freie Wirkende (14) des Instruments sowie den Zielpunkt (16) im Körper (17) behält.

2. Positioniersystem nach Anspruch 1, **dadurch gekennzeichnet, daß** das Innengewindeteil (9) und die Gewindespindel (7) selbsthemmend ausgeführt sind.

3. Positioniersystem nach einem der vorhergehenden Ansprüche, **dadurch gekennzeichnet, daß** das Positioniersystem aufgrund der Freiheitsgrade der Linearführung (5) und des Kugelgelenks (3) zusammengefaßt für eine axiale (15) und drei rotatorische (22, 23, 24) Bewegungen des Instrumentes (12) und seines freien Wirkendes (14) in Körperöffnungen (28) des Menschen ausgelegt ist.

4. Positioniersystem nach einem der vorhergehenden Ansprüche, ferner versehen mit einem mit der Gewindespindel (7) verbundenen Drehknauf (8) zum axialen und selbsthemmenden Verfahren der Aufnahmevorrichtung (6, 11) durch manuelles Drehen an dem Drehknauf.

5. Positioniersystem nach einem der vorhergehenden Ansprüche, ferner versehen mit einer Klemmschraube (20) zum Fixieren aller drei rotatorischen Freiheitsgrade (22, 23, 24) des Kugelgelenks (3) in ihrer aktuellen Lage.

6. Positioniersystem nach einem der vorhergehenden Ansprüche, **dadurch gekennzeichnet, daß** die aktuelle Position der rotatorischen Freiheitsgrade (22, 23, 24) bei gelöster Klemmung des Kugelgelenks (3) durch Reibkräfte gesichert ist und nach dem Schließen der Klemmung (19, 20, 26) dauerhaft beibehalten wird.

7. Positioniersystem nach einem der vorhergehenden Ansprüche, **dadurch gekennzeichnet, daß** es sich bei dem Instrument (12) um einen elektromechanischen Aktor (12) zur vibratorischen Stimulation der Gehörknöchelchenkette (Hammer, Amboß, Steigbügel) oder des Trommelfells handelt und das Positioniersystem zum Positionieren und Ankoppeln des elektromechanischen Aktors (12) an Hammer, Amboß, Steigbügel oder Trommelfell als körperfestem Zielpunkt (16) und zum Fixieren des Aktors in dieser Lage ausgelegt ist.

8. Positioniersystem nach einem der vorhergehenden Ansprüche, **dadurch gekennzeichnet, daß** die Halterung einen Kopfhalter (31) aufweist.

9. Positioniersystem nach Anspruch 8, **dadurch gekennzeichnet, daß** der Kopfhalter (31) auf seiner Arbeitsseite (37) mit einem trichterförmigen Halteelement (36) zur Fixierung im Gehörgang versehen ist, und das freie Wirkende (14) des Instrumentes (12) zum Zielort (16) im Körper (17) durch das trichterförmige Halteelement hindurchführbar ist.

10. Positioniersystem nach Anspruch 8 oder 9, **dadurch gekennzeichnet, daß** der Kopfhalter (31) zur beidseitigen schmerzfreien Befestigung im Ohrbereich des Schädels ausgebildet ist.

11. Positioniersystem nach einem der Ansprüche 8 bis 10, **dadurch gekennzeichnet, daß** der Kopfhalter (31) auf der der Arbeitsseite (37) gegenüberliegenden Seite (39) ein die Ohrmuschel umschließendes, weiches Formteil (38) aufweist, das als zweite Auflagefläche für den Kopfhalter dient und insbesondere als einseitiger Kopfhörer oder Schallschutz ausgebildet ist.

12. Positioniersystem nach einem der Ansprüche 8 bis 11, **dadurch gekennzeichnet, daß** ein Gewindetrieb und ein Drehknauf (33) zum stufenlosen und selbsthemmenden Verstellen der Einspannweite (32) des Kopfhalters (31) zwischen Arbeitsseite (37) und Gegenseite (39) vorgesehen sind.

## Claims

1. Fixable positioning system for secure, play-free fastening to the human skull, in particular for the non-invasive coupling of an actuator or a sensor instrument to the external side of the ear membrane, comprising:
- a holder (27, 31) which can be secured to the skull;
- a base (4) attached to the holder (27, 31);
- a linear drive mechanism (2) having a linear guide (5) and an internally threaded member (9) attached thereto as well as a threaded spindle (7) which is manually rotatable, the axis of said threaded spindle being oriented in parallel to the linear guide (5);
- a receiving device (6, 11) for a therapeutic or diagnostic instrument (12), said receiving device (6, 11) being guided in said linear guide (5);
- a ball-and-socket joint (3) which is disposed between the base (4) and the linear guide (5) and which may be manually positioned and then fixed via a clamp mechanism (20, 26, 42);
- wherein the longitudinal axis of the instrument (12) is laterally offset with respect to the axis of the linear guide (5) and to the ball-and-socket joint (3), and wherein the construction and the geometrical dimensions of the positioning system are configured such that the operator always maintains an unobstructed view of at least the free active end (14) of the instrument as well as of the destination (16) within the body (17), regardless of whether observation takes place by the naked eye or by using a microscope.

2. Positioning system according to claim 1, **characterized in that** the internally threaded member (9) and the threaded spindle (7) have a self-locking construction.

3. Positioning system according to any one of the preceding claims, **characterized in that** the positioning system, as a result of the degrees of freedom of the linear guide (5) and of the ball-and-socket joint (3), is designed for permitting in combination one axial (15) and three rotational (22, 23, 24) movements of the instrument (12) and of its free active end (14) within body openings (28) of a human being.

4. Positioning system according to any one of the preceding claims, further comprising a rotary knob (8) connected to the threaded spindle (7) for axial and self-locking movement of the receiving device (6, 11) by manually turning the rotary knob.

5. Positioning system according to any one of the preceding claims, further comprising a clamp screw (20) for fixing all three rotational degrees of freedom (22, 23, 24) of the ball-and-socket joint (3) in their current position.

6. Positioning system according to any one of the preceding claims, wherein the current position of the rotational degrees of freedom (22, 23, 24) is secured by frictional forces when said ball-and-socket joint (3) is in a released condition and is permanently maintained after closing of the clamp mechanism (19, 20, 26).

7. Positioning system according to any one of the preceding claims, **characterized in that** said instrument (12) is an electromechanical actuator (12) for vibrational stimulation of the ossicular chain (malleus, incus, stapes) or of the eardrum, and that the positioning system is adapted for positioning and coupling of said electromechanical actuator (12) to malleus, incus, stapes or eardrum as destination (16) within the body and for fixing of said actuator in this position.

8. Positioning system according to any one of the preceding claims, **characterized in that** said holder comprises a head support (31).

9. Positioning system according to claim 8, **characterized in that** said head support (31), on its working side (37), is provided with a funnel-shaped retaining element (36) for fixation in the auditory canal, said free, active end (14) of said instrument (12) being able to pass through the funnel-shaped retaining element to the destination (16) in the body (17).

10. Positioning system according to claim 8 or 9, **characterized in that** the head support (31) is adapted for bilateral, painless attachment in the ear area of the skull.

11. Positioning system according to any one of claims 8 to 10, **characterized in that** the head support (31), on the side (39) opposite its working side (37), comprises a soft molded part (38) which surrounds the outer ear and which forms a second bearing surface for the head support and which particularly is made as a one-sided headset or noise protector.

12. Positioning system according to anyone of claims 8 to 11, **characterized in that** there are provided a threaded drive and a rotary knob (33) for continuous and self-locking adjustment of the clamping width (32) of the head support (31) between the working side (37) and the opposite side (39).

## Revendications

1. Système de positionnement adapté à se fixer, destiné à être attaché fermement et sans jeu contre le crâne humain, en particulier destiné au raccordement non invasif d'un instrument d'acteur ou sensoriel contre la face extracorporelle de la membrane du tympan, comprenant
- un support (27, 31) adapté à se fixer contre le crâne ;
- une base (4) montée contre le support (27, 31) ;
- un mécanisme d'entraînement linéaire (2), qui comporte un guidage linéaire (5) muni d'une pièce taraudée (9), ainsi qu'une broche filetée (7) disposée parallèlement à l'axe du guidage linéaire (5) et susceptible d'être entraînée en rotation manuellement par rapport au guidage linéaire ;
- un dispositif à logement (6, 11) guidé contre le guidage linéaire (5) et destiné à recevoir un instrument (12) de thérapie ou de diagnostic comportant une broche longitudinale ; et
- un joint à rotule (3) disposé entre la base (4) et le guidage linéaire (5), susceptible d'être positionné manuellement et d'être bloqué par un mécanisme de serrage (20, 26, 42) ;
- dans lequel la broche longitudinale de l'instrument (12) est décalée latéralement par rapport à l'axe du guidage linéaire (5) et par rapport au joint à rotule (3) et dans lequel la construction et les dimensions géométriques du système de positionnement sont choisies de telle sorte que la personne chargée de l'intervention garde toujours une vue dégagée, soit à l'oeil nu, soit à l'aide d'un microscope, au moins sur l'extrémité active (14) libre de l'instrument, ainsi que sur le point cible (16) dans le corps humain (17).

2. Système de positionnement selon la revendication 1, **caractérisé en ce que** la pièce taraudée (9) et la broche filetée (7) sont conçues avec un blocage automatique.

3. Système de positionnement selon une des revendications précédentes, **caractérisé en ce que** le système de positionnement, en raison des degrés de liberté du guidage linéaire (5) et du joint à rotule (3), est conçu globalement pour un mouvement axial (15) et trois mouvements rotatoires (22, 23, 24) de l'instrument (12) et de son extrémité active (14) libre dans des orifices (28) du corps humain.

4. Système de positionnement selon une des revendications précédentes, muni en outre d'un bouton rotatif (8) relié à la broche filetée (7) pour le déplacement axial et autobloquant du dispositif à logement (6, 11) par la rotation manuelle du bouton rotatif.

5. Système de positionnement selon une des revendications précédentes, muni en outre d'une vis de blocage (20) destinée à bloquer le joint à rotule (3) dans sa position actuelle dans chacun des trois degrés de liberté rotatoires (22, 23, 24).

6. Système de positionnement selon une des revendications précédentes, **caractérisé en ce que**, au cas où le système de blocage du joint à rotule (3) serait desserré, la position actuelle des degrés de liberté rotatoires (22, 23, 24) est garantie par des forces de frottement et elle est maintenue en permanence après la fermeture du système de blocage (19, 20, 26).

7. Système de positionnement selon une des revendications précédentes, **caractérisé en ce que** l'instrument (12) est un acteur électromécanique (12) pour la stimulation vibratoire de la chaîne ossiculaire (marteau, enclume, étrier) ou de la membrane du tympan et le système de positionnement est conçu pour positionner et raccorder l'acteur électromécanique (12) avec le marteau, l'enclume et l'étrier ou la membrane du tympan formant le point cible (16) fixe dans le corps humain et pour bloquer l'acteur dans cette position.

8. Système de positionnement selon une des revendications précédentes, **caractérisé en ce que** le support est un support de tête (31).

9. Système de positionnement selon la revendication 8, **caractérisé en ce que** le support de tête (31) est muni sur sa face dite de travail (37) d'un élément de maintien (36) en forme d'entonnoir, destiné à être bloqué dans le conduit auditif, et l'extrémité active (14) libre de l'instrument (12) peut être guidée vers le point cible (16) dans le corps humain (17) en passant à travers l'élément de maintien en forme d'entonnoir.

10. Système de positionnement selon la revendication 8 ou 9, **caractérisé en ce que** le support de tête (31) est conçu pour être fixé sans douleur de part et d'autre du crâne dans la région de l'oreille.

11. Système de positionnement selon une des revendications 8 à 10, **caractérisé en ce que** le support de tête (31) sur la face (39) opposée à la face de travail (37) comporte une pièce moulée (38) enveloppant le pavillon de l'oreille, laquelle est destinée à former une deuxième surface de contact du support de tête et est conçue en particulier sous forme d'écouteur unilatéral ou sous forme de protection acoustique.

12. Système de positionnement selon une des revendications 8 à 11, **caractérisé en ce qu'**un mécanisme d'entraînement fileté et un bouton rotatif (33) sont prévus pour le réglage progressif et autobloquant de la largeur libre (32) entre la face de travail (37) et la face opposée (39) du support de tête (31).
